# EUROPEAN PATENT APPLICATION

(11) **EP 4 321 100 A1**
(43) Date of publication of application: **14.02.2024**
(21) Application number: 22784515.3
(22) Date of filing: 23.03.2022
(51) Int. Cl.: A61B 6/00, A61B 6/03, G16H 30/20

(54) **MEDICAL IMAGE DEVICE, MEDICAL IMAGE METHOD, AND MEDICAL IMAGE PROGRAM**

(30) Priority: 07.04.2021 JP 2021065375; 22.12.2021 JP 2021208525
(71) Applicant: FUJIFILM Corporation, Tokyo 106-8620 (JP)
(72) Inventor: ICHINOSE, Akimichi, Tokyo 106-8620 (JP); NAKAMURA, Keigo, Tokyo 106-8620 (JP)
(74) Representative: Hughes, Andrea Michelle
(86) International application number: PCT/JP2022/013693
(87) International publication number: WO 2022/215530

(57) **Abstract**

A medical image apparatus acquires a medical image, information indicating a plurality of regions of interest included in the medical image, and an attribute of each of the plurality of regions of interest, selects at least one region of interest from among the plurality of regions of interest, and performs control to display information regarding a region of interest other than the selected region of interest based on an attribute of the selected region of interest.

## Description

### BACKGROUND OF THE INVENTION

### 1. Field of the Invention

The present disclosure relates to a medical image apparatus, a medical image method, and a medical image program.

### 2. Description of the Related Art

WO2020/209382A discloses a technology of detecting a plurality of findings representing features of an abnormal shadow included in a medical image, specifying at least one finding to be used for creating an interpretation report from among the detected findings, and creating an interpretation report using the specified finding.

### SUMMARY OF THE INVENTION

Incidentally, in the technology disclosed in WO2020/209382A, in a case where a medical image includes a large number of regions of interest, a doctor has to designate each region of interest to create a medical document such as an interpretation report, which has been troublesome for the doctor. That is, the technology disclosed in WO2020/209382A may not be able to appropriately support the creation of a medical document in a case where a medical image includes a large number of regions of interest.

The present disclosure has been made in view of the above circumstances, and an object of the present disclosure is to provide a medical image apparatus, a medical image method, and a medical image program capable of appropriately supporting the creation of a medical document even in a case where a medical image includes a large number of regions of interest.

According to an aspect of the present disclosure, there is provided a medical image apparatus comprising: at least one processor, in which the processor is configured to: acquire a medical image, information indicating a plurality of regions of interest included in the medical image, and an attribute of each of the plurality of regions of interest; select at least one region of interest from among the plurality of regions of interest; and perform control to display information regarding a region of interest other than the selected region of interest based on an attribute of the selected region of interest.

In addition, in the medical image apparatus according to the aspect of the present disclosure, the processor may be configured to perform control to display information regarding a region of interest having the same attribute as the attribute of the selected region of interest.

In addition, the medical image apparatus according to the present disclosure, the processor may be configured to perform control to display information regarding a region of interest having an attribute different from the attribute of the selected region of interest.

In addition, in the medical image apparatus according to the aspect of the present disclosure, the processor may be configured to, in a case where the number of regions of interest having the same attribute as the attribute of the selected region of interest is equal to or greater than a threshold value, perform control to display the information regarding the region of interest having the attribute different from the attribute of the selected region of interest.

In addition, in the medical image apparatus according to the aspect of the present disclosure, the region of interest may be a region including a lesion, the attribute may include whether the lesion is benign or malignant, and the processor may be configured to, in a case where the selected region of interest includes a benign lesion and the number of regions of interest including the benign lesion is equal to or greater than the threshold value, perform control to display information regarding a region of interest including a malignant lesion.

In addition, in the medical image apparatus according to the aspect of the present disclosure, the processor may be configured to perform control to highlight the region of interest as the control to display the information regarding the region of interest.

In addition, in the medical image apparatus according to the aspect of the present disclosure, the processor may be configured to perform control to display information indicating a presence of the region of interest having the attribute different from the attribute of the selected region of interest as the control to the display information regarding the region of interest.

In addition, in the medical image apparatus according to the aspect of the present disclosure, the processor may be configured to, in a case where an attribute of the region of interest other than the selected region of interest is different from an attribute detected in the past, perform control to further display information indicating that the attributes are different.

In addition, according to another aspect of the present disclosure, there is provided a medical image method executed by a processor provided in a medical image apparatus, the method comprising: acquiring a medical image, information indicating a plurality of regions of interest included in the medical image, and an attribute of each of the plurality of regions of interest; selecting at least one region of interest from among the plurality of regions of interest; and performing control to display information regarding a region of interest other than the selected region of interest based on an attribute of the selected region of interest.

In addition, according to another aspect of the present disclosure, there is provided a medical image program for causing a processor provided in a medical image apparatus to execute: acquiring a medical image, information indicating a plurality of regions of interest included in the medical image, and an attribute of each of the plurality of regions of interest; selecting at least one region of interest from among the plurality of regions of interest; and performing control to display information regarding a region of interest other than the selected region of interest based on an attribute of the selected region of interest.

In addition, according to another aspect of the present disclosure, there is provided a medical image apparatus comprising: at least one processor, in which the processor is configured to: acquire a medical image, information indicating a plurality of regions of interest included in the medical image, and an attribute of each of the plurality of regions of interest; select at least one region of interest from among the plurality of regions of interest; and generate a comment on findings for a region of interest having the same attribute as an attribute of the selected region of interest.

In addition, according to another aspect of the present disclosure, there is provided a medical image method executed by a processor provided in a medical image apparatus, the method comprising: acquiring a medical image, information indicating a plurality of regions of interest included in the medical image, and an attribute of each of the plurality of regions of interest; selecting at least one region of interest from among the plurality of regions of interest; and generating a comment on findings for a region of interest having the same attribute as an attribute of the selected region of interest.

In addition, according to another aspect of the present disclosure, there is provided a medical image program for causing a processor provided in a medical image apparatus to execute: acquiring a medical image, information indicating a plurality of regions of interest included in the medical image, and an attribute of each of the plurality of regions of interest; selecting at least one region of interest from among the plurality of regions of interest; and generating a comment on findings for a region of interest having the same attribute as an attribute of the selected region of interest.

According to the aspects of the present disclosure, it is possible to appropriately support the creation of a medical document even in a case where a medical image includes a large number of regions of interest.

### BRIEF DESCRIPTION OF THE DRAWINGS

Fig. 1 is a block diagram showing a schematic configuration of a medical information system.
Fig. 2 is a block diagram showing an example of a hardware configuration of a medical image apparatus.
Fig. 3 is a block diagram showing an example of a functional configuration of a medical image apparatus according to first and second embodiments.
Fig. 4 is a diagram for describing a process of extracting a lesion.
Fig. 5 is a diagram for describing a process of deriving a name of a lesion.
Fig. 6 is a diagram showing an example of a screen in which a lesion is highlighted.
Fig. 7 is a flowchart showing an example of a lesion display process according to the first embodiment.
Fig. 8 is a diagram for describing a process of deriving an attribute of a lesion.
Fig. 9 is a diagram showing an example of a screen in which a lesion is highlighted.
Fig. 10 is a flowchart showing an example of a lesion display process according to the second embodiment.
Fig. 11 is a block diagram showing an example of a functional configuration of a medical image apparatus according to a third embodiment.
Fig. 12 is a diagram for describing a process of deriving a name and a finding of a lesion.
Fig. 13 is a diagram for describing a process of generating a comment on findings.
Fig. 14 is a flowchart showing an example of a comment-on-findings generation process according to the third embodiment.
Fig. 15 is a diagram showing an example of a screen in which a lesion is highlighted according to a modification example.

### DESCRIPTION OF THE PREFERRED EMBODIMENTS

Hereinafter, form examples for implementing a technology of the present disclosure will be described in detail with reference to the drawings.

### [First embodiment]

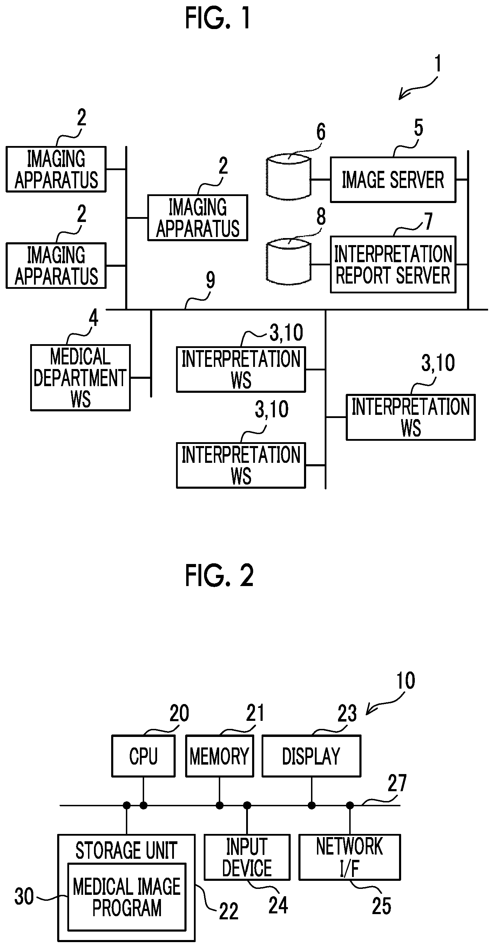

First, a configuration of a medical information system 1 to which a medical image apparatus according to the disclosed technology is applied will be described with reference to Fig. 1. The medical information system 1 is a system for performing imaging of a diagnosis target part of a subject and storing of a medical image acquired by the imaging based on an examination order from a doctor in a medical department using a known ordering system. In addition, the medical information system 1 is a system for performing interpretation of a medical image and creation of an interpretation report by a radiologist, and viewing the interpretation report and detailed observation of the medical image to be interpreted by a doctor of a medical department that is a request source.

As shown in Fig. 1, the medical information system 1 according to the present embodiment includes a plurality of imaging apparatuses 2, a plurality of interpretation workstations (WS) 3 that are interpretation terminals, a medical department WS 4, an image server 5, an image database (DB) 6, an interpretation report server 7, and an interpretation report DB 8. The imaging apparatus 2, the interpretation WS 3, the medical department WS 4, the image server 5, and the interpretation report server 7 are connected to each other via a wired or wireless network 9 in a communicable state. In addition, the image DB 6 is connected to the image server 5, and the interpretation report DB 8 is connected to the interpretation report server 7.

The imaging apparatus 2 is an apparatus that generates a medical image showing a diagnosis target part of a subject by imaging the diagnosis target part. The imaging apparatus 2 may be, for example, a simple X-ray imaging apparatus, an endoscope apparatus, a computed tomography (CT) apparatus, a magnetic resonance imaging (MRI) apparatus, a positron emission tomography (PET) apparatus, and the like. A medical image generated by the imaging apparatus 2 is transmitted to the image server 5 and is saved therein.

The medical department WS 4 is a computer used by a doctor in the medical department for detailed observation of a medical image, viewing of an interpretation report, creation of an electronic medical record, and the like. In the medical department WS 4, each process such as creating an electronic medical record of a patient, requesting the image server 5 to view an image, and displaying a medical image received from the image server 5 is performed by executing a software program for each process. In addition, in the medical department WS 4, each process such as automatically detecting or highlighting suspected disease regions in the medical image, requesting to view an interpretation report from the interpretation report server 7, and displaying the interpretation report received from the interpretation report server 7 is performed by executing a software program for each process.

The image server 5 incorporates a software program that provides a function of a database management system (DBMS) to a general-purpose computer. In a case where the image server 5 receives a request to register a medical image from the imaging apparatus 2, the image server 5 prepares the medical image in a format for a database and registers the medical image in the image DB 6.

Image data representing the medical image acquired by the imaging apparatus 2 and accessory information attached to the image data are registered in the image DB 6. The accessory information includes information such as an image identification (ID) for identifying individual medical images, a patient ID for identifying a patient who is a subject, an examination ID for identifying examination content, and a unique identification (UID) assigned to each medical image, for example. In addition, the accessory information includes information such as an examination date when a medical image was generated, an examination time, the type of imaging apparatus used in the examination for acquiring the medical image, patient information (for example, a name, an age, and a gender of the patient), an examination part (that is, an imaging part), and imaging information (for example, an imaging protocol, an imaging sequence, an imaging method, imaging conditions, and whether or not a contrast medium is used), and a series number or collection number when a plurality of medical images are acquired in one examination. In addition, in a case where a viewing request from the interpretation WS 3 is received through the network 9, the image server 5 searches for a medical image registered in the image DB 6 and transmits the searched for medical image to the interpretation WS 3 that is a request source.

The interpretation report server 7 incorporates a software program for providing a function of DBMS to a general-purpose computer. In a case where the interpretation report server 7 receives a request to register an interpretation report from the interpretation WS 3, the interpretation report server 7 prepares the interpretation report in a format for a database and registers the interpretation report in the interpretation report database 8. Further, in a case where the request to search for the interpretation report is received, the interpretation report is searched for from the interpretation report DB 8.

In the interpretation report DB 8, for example, an interpretation report is registered in which information, such as an image ID for identifying a medical image to be interpreted, a radiologist ID for identifying an image diagnostician who performed the interpretation, a lesion name, position information of a lesion, findings, and a degree of certainty of the findings, is recorded.

The network 9 is a wired or wireless local area network that connects various apparatuses in a hospital to each other. In a case where the interpretation WS 3 is installed in another hospital or clinic, the network 9 may be configured to connect local area networks of respective hospitals through the Internet or a dedicated line. In any case, it is preferable that the network 9 has a configuration capable of realizing high-speed transmission of medical images such as an optical network.

The interpretation WS 3 requests the image server 5 to view a medical image, performs various types of image processing on the medical image received from the image server 5, displays the medical image, performs an analysis process on the medical image, highlights the medical image based on an analysis result, and creates an interpretation report based on the analysis result. In addition, the interpretation WS 3 supports creation of an interpretation report, requests the interpretation report server 7 to register and view an interpretation report, displays the interpretation report received from the interpretation report server 7, and the like. The interpretation WS 3 performs each of the above processes by executing a software program for each process. The interpretation WS 3 encompasses a medical image apparatus 10 to be described later, and in the above processes, processes other than those performed by the medical image apparatus 10 are performed by a well-known software program, and therefore the detailed description thereof will be omitted here. In addition, processes other than the processes performed by the medical image apparatus 10 may not be performed in the interpretation WS 3, and a computer that performs the processes may be separately connected to the network 9, and in response to a processing request from the interpretation WS 3, the requested process may be performed by the computer. Hereinafter, the medical image apparatus 10 encompassed in the interpretation WS 3 will be described in detail.

Next, a hardware configuration of the medical image apparatus 10 according to the present embodiment will be described with reference to Fig. 2. As shown in Fig. 2, the medical image apparatus 10 includes a central processing unit (CPU) 20, a memory 21 as a temporary storage area, and a non-volatile storage unit 22. Further, the medical image apparatus 10 includes a display 23 such as a liquid crystal display, an input device 24 such as a keyboard and a mouse, and a network interface (I/F) 25 connected to the network 9. The CPU 20, the memory 21, the storage unit 22, the display 23, the input device 24, and the network I/F 25 are connected to a bus 27.

The storage unit 22 is realized by a hard disk drive (HDD), a solid state drive (SSD), a flash memory, or the like. A medical image program 30 is stored in the storage unit 22 as a storage medium. The CPU 20 reads out the medical image program 30 from the storage unit 22, loads the read medical image program 30 into the memory 21, and executes the loaded medical image program 30.

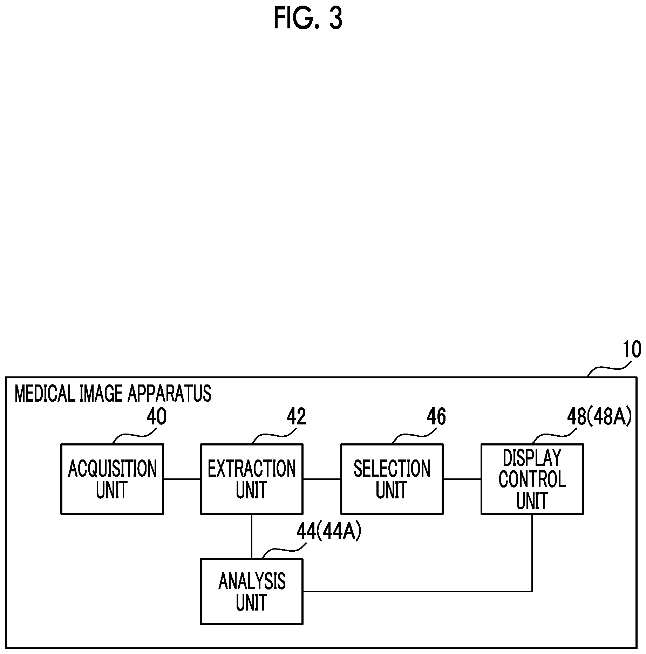

Next, a functional configuration of the medical image apparatus 10 according to the present embodiment will be described with reference to Fig. 3. As shown in Fig. 3, the medical image apparatus 10 includes an acquisition unit 40, an extraction unit 42, an analysis unit 44, a selection unit 46, and a display control unit 48. The CPU 20 executes the medical image program 30 to function as the acquisition unit 40, the extraction unit 42, the analysis unit 44, the selection unit 46, and the display control unit 48.

The acquisition unit 40 acquires a medical image to be diagnosed (hereinafter referred to as a "diagnosis target image") from the image server 5 via the network I/F 25. In the following, a case where the diagnosis target image is a CT image of the liver will be described as an example.

The extraction unit 42 extracts a region including a lesion from the diagnosis target image acquired by the acquisition unit 40. Specifically, the extraction unit 42 extracts a region including a lesion using a trained model M1 for detecting the lesion from the diagnosis target image. A region including a lesion in a diagnosis target image is an example of a region of interest according to the disclosed technology. The region of interest is not limited to a region including a lesion, and regions of organs such as the lung and the liver may be applied, or regions of an anatomical structure such as subsegments divided into S1 to S8 of the liver may be applied.

The trained model M1 is configured by, for example, a convolutional neural network (CNN) that receives a medical image as an input and outputs a region including a lesion included in the medical image. The trained model M1 is, for example, a model trained by machine learning using, as training data, a large number of combinations of a medical image including a lesion and information specifying a region in the medical image in which the lesion is present.

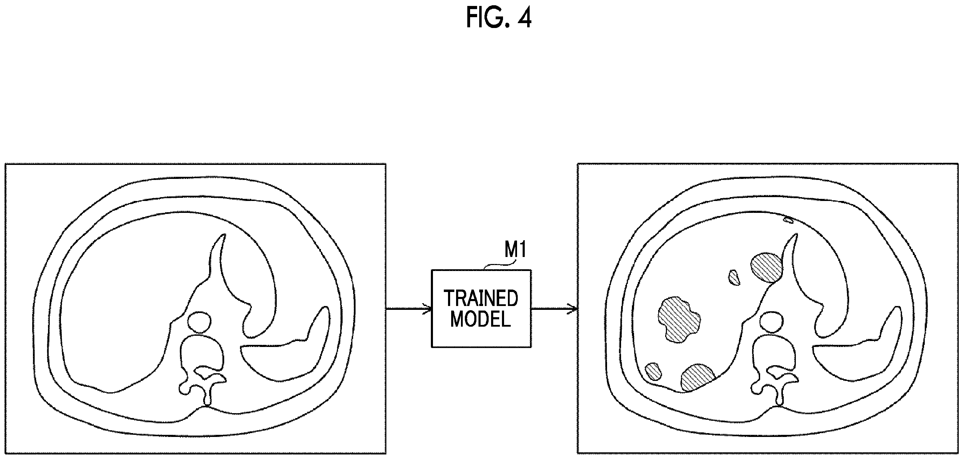

As shown in Fig. 4 as an example, the extraction unit 42 inputs the diagnosis target image to the trained model M1. The trained model M1 outputs information specifying a region in which a lesion included in the input diagnosis target image is present. In the example of Fig. 4, the region filled with the diagonal line indicates the lesion. In addition, the extraction unit 42 may extract a region including a lesion by a known computer-aided diagnosis (CAD), or may extract a region designated by the user as a region including the lesion.

The analysis unit 44 analyzes each of the lesions extracted by the extraction unit 42, and derives a name of the lesion as an example of attributes of the lesion. Specifically, the analysis unit 44 derives a name of the lesion using a trained model M2 for deriving the name of the lesion. The trained model M2 is configured by, for example, a CNN that receives, for example, a medical image including a lesion and information specifying a region in the medical image in which the lesion is present as inputs, and outputs a name of the lesion. The trained model M2 is, for example, a model trained by machine learning using, as training data, a large number of combinations of information specifying a medical image including a lesion and a region in the medical image in which the lesion is present, and a name of the lesion.

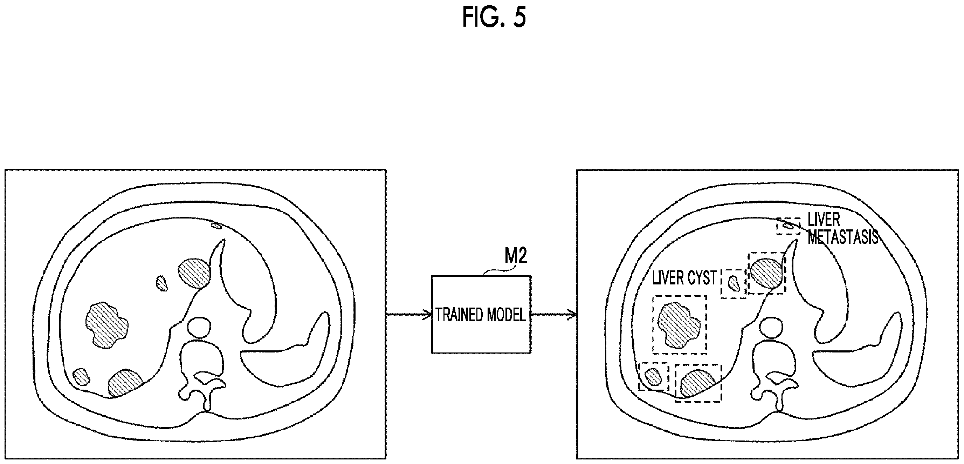

As shown in Fig. 5 as an example, the analysis unit 44 inputs, to the trained model M2, information specifying a diagnosis target image and a region in which the lesion extracted by the extraction unit 42 for the diagnosis target image is present. The trained model M2 outputs the name of the lesion included in the input diagnosis target image. Fig. 5 shows an example in which the name of five lesions is liver cyst and the name of one lesion is liver metastasis. Note that the attribute of the lesion is not limited to the name of the lesion, and may be, for example, findings such as a position, a size, the presence or absence of calcification, whether the lesion is benign or malignant, and the presence or absence of an irregular margin. Further, a plurality of attributes of the lesion may be used.

The selection unit 46 selects at least one lesion designated by the user from among a plurality of lesions extracted by the extraction unit 42.

The display control unit 48 acquires, from the extraction unit 42, information indicating the plurality of lesions included in the diagnosis target image extracted by the extraction unit 42. In addition, the display control unit 48 acquires, from the analysis unit 44, the attribute of each of the plurality of lesions derived by the analysis unit 44. In addition, the display control unit 48 may acquire, from an external device such as the medical department WS 4, information indicating the plurality of lesions included in the diagnosis target image and an attribute of each of the plurality of lesions. In this case, the extraction unit 42 and the analysis unit 44 are provided by the external device.

The display control unit 48 performs control to display information indicating the plurality of lesions extracted by the extraction unit 42 on the display 23. The user designates a lesion for which a medical document such as an interpretation report is to be created from among the plurality of lesions displayed on the display 23. This designated lesion is selected by the selection unit 46 described above.

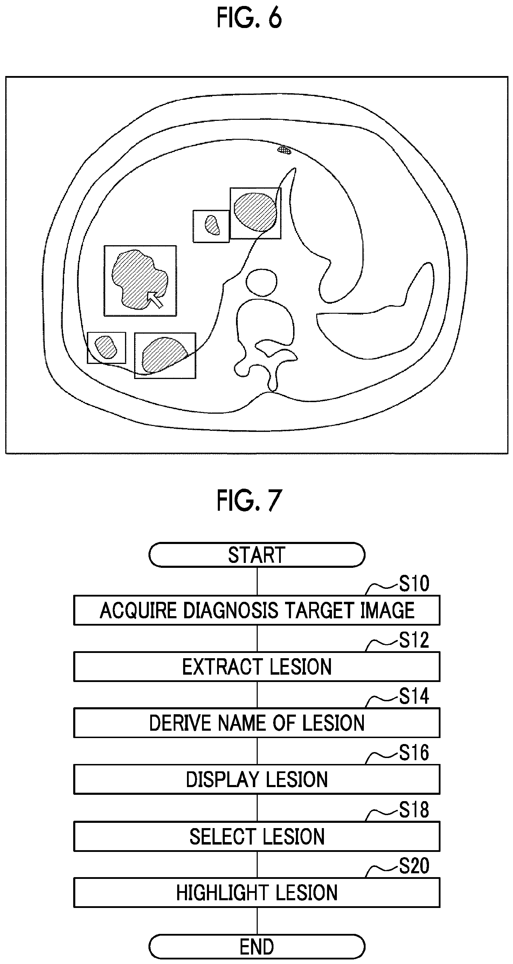

In addition, based on an attribute of a lesion selected by the selection unit 46 (hereinafter referred to as a "first lesion"), the display control unit 48 performs control to display information regarding lesions other than the first lesion (hereinafter referred to as "second lesions") on the display 23. In the present embodiment, the display control unit 48 performs control to highlight the first lesion and the lesion having the same name as the first lesion among the second lesions on the display 23. As shown in Fig. 6 as an example, the display control unit 48 performs control to highlight lesions by surrounding the first lesion and the lesion having the same name as the first lesion with a rectangular frame line. Fig. 6 shows an example of highlighting in a case where one of the lesions of the liver cyst in Fig. 5 (in the example of Fig. 6, the lesion pointed to by the arrow indicating the mouse pointer) is designated by the user. In this way, the user can easily ascertain the lesion having the same name as the lesion designated by the user as the creation target of the medical document. Accordingly, the user can easily create a comment on findings summarizing the findings of the lesions having the same name.

In addition, the display control unit 48 may perform control to display the name of the lesion in an identifiable manner by setting the color of the frame line to a color preset according to the name of the lesion. Further, for example, the display control unit 48 may perform control to highlight the lesion by blinking the lesion, adding a predetermined mark, drawing an outer edge of the region of the lesion with a line, or the like.

Next, with reference to Fig. 7, operations of the medical image apparatus 10 according to the present embodiment will be described. The CPU 20 executes the medical image program 30, whereby a lesion display process shown in Fig. 7 is executed. The lesion display process shown in Fig. 7 is executed, for example, in a case where an instruction to start execution is input by the user.

In Step S10 of Fig. 7, the acquisition unit 40 acquires the diagnosis target image from the image server 5 via the network I/F 25. In Step S12, as described above, the extraction unit 42 extracts a region including a lesion from the diagnosis target image acquired in Step S10. In Step S14, as described above, the analysis unit 44 analyzes each of the lesions extracted in Step S12, and derives a name of the lesion.

In Step S16, the display control unit 48 performs control to display information indicating the plurality of lesions extracted in Step S12 on the display 23. The user designates a lesion for which a medical document such as an interpretation report is to be created from among the plurality of lesions displayed on the display 23. In Step S18, the selection unit 46 selects at least one lesion designated by the user from among the plurality of lesions.

In Step S20, as described above, the display control unit 48 performs control to highlight the first lesion selected in Step S18 and the lesion having the same name as the first lesion on the display 23. In a case where the process of Step S20 ends, the lesion display process ends.

As described above, according to the present embodiment, even in a case where a medical image includes a large number of lesions, lesions having the same attribute as the lesion designated by the user are highlighted, and thus the user can easily create a medical document. Therefore, it is possible to appropriately support the creation of the medical document.

### [Second embodiment]

A second embodiment of the disclosed technology will be described. Since the configuration of the medical information system 1 and the hardware configuration of the medical image apparatus 10 according to the present embodiment are the same as those of the first embodiment, the description thereof will be omitted.

A functional configuration of the medical image apparatus 10 according to the present embodiment will be described with reference to Fig. 3. The same reference numerals are assigned to the functional units having the same functions as the medical image apparatus 10 according to the first embodiment, and the description thereof will be omitted. As shown in Fig. 3, the medical image apparatus 10 includes an acquisition unit 40, an extraction unit 42, an analysis unit 44A, a selection unit 46, and a display control unit 48A. The CPU 20 executes the medical image program 30 to function as the acquisition unit 40, the extraction unit 42, the analysis unit 44A, the selection unit 46, and the display control unit 48A.

The analysis unit 44A analyzes each of the lesions extracted by the extraction unit 42, and derives whether the lesion is benign or malignant as an example of attributes of the lesion. Specifically, the analysis unit 44A derives whether the lesion is benign or malignant using a trained model M3 for deriving whether the lesion is benign or malignant. The trained model M3 is configured by, for example, a CNN that receives, for example, a medical image including a lesion and information specifying a region in the medical image in which the lesion is present as inputs, and outputs whether the lesion is benign or malignant. The trained model M3 is, for example, a model trained by machine learning using, as training data, information specifying a medical image including a lesion and a region in the medical image in which the lesion is present, and information indicating whether the lesion is benign or malignant.

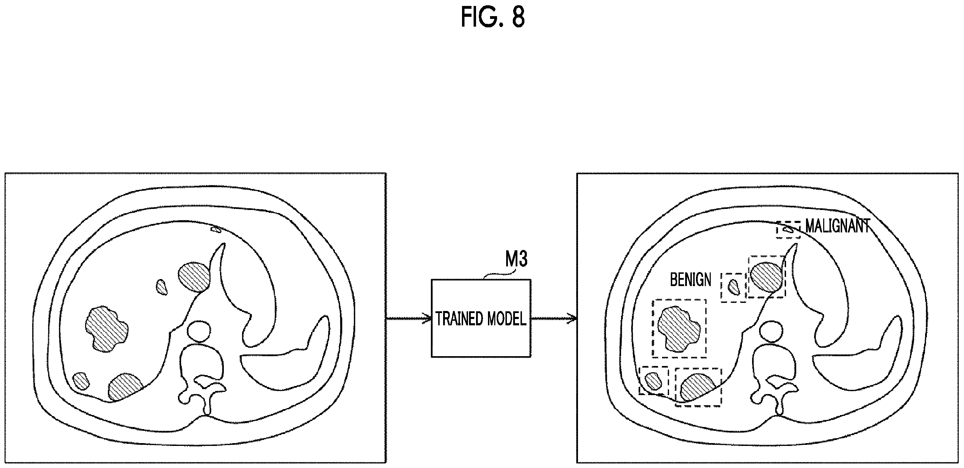

As shown in Fig. 8 as an example, the analysis unit 44A inputs, to the trained model M3, information specifying a diagnosis target image and a region in which a lesion extracted by the extraction unit 42 for the diagnosis target image is present. The trained model M3 outputs whether a lesion included in the input diagnosis target image is benign or malignant. Fig. 8 shows an example in which five lesions are benign and one lesion is malignant.

Similarly to the display control unit 48 according to the first embodiment, the display control unit 48A performs control to display information indicating the plurality of lesions extracted by the extraction unit 42 on the display 23.

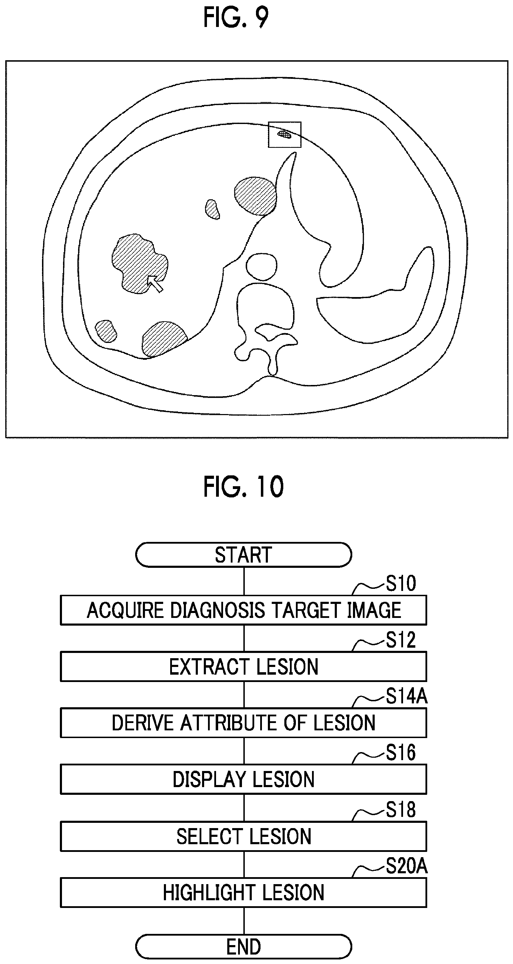

In addition, the display control unit 48A performs control to display information regarding the second lesion other than the first lesion on the display 23 based on the attribute of the first lesion selected by the selection unit 46. In the present embodiment, the display control unit 48A performs control to highlight a lesion having an attribute different from the attribute of the first lesion among the second lesions on the display 23. Since the highlighting method is the same as that of the first embodiment, detailed description thereof will be omitted. As shown in Fig. 9 as an example, a lesion having an attribute different from that of the lesion designated by the user is highlighted under the control by the display control unit 48A. Fig. 9 shows an example of highlighting in a case where one of the benign lesions in Fig. 8 (in the example of Fig. 9, the lesion pointed to by the arrow indicating the mouse pointer) is designated by the user.

In a case where the number of lesions having the same attribute as the attribute of the first lesion selected by the selection unit 46 is equal to or greater than a threshold value TH, the display control unit 48A may perform control to highlight, on the display 23, a lesion having an attribute different from the attribute of the first lesion. Specifically, in a case where the attribute of the first lesion selected by the selection unit 46 is a benign lesion and the number of benign lesions is equal to or greater than the threshold value TH, the display control unit 48A may perform control to highlight a malignant lesion on the display 23. Thereby, it is possible to suppress overlooking of malignant lesions by the user due to the presence of a large number of benign lesions in the medical image.

In addition, in a case where the number of lesions having the same attribute as the attribute of the first lesion selected by the selection unit 46 is equal to or greater than the threshold value TH, the display control unit 48A may perform control to display, on the display 23, information indicating a presence of a lesion having an attribute different from the attribute of the first lesion. Specifically, in a case where the attribute of the first lesion selected by the selection unit 46 is a benign lesion and the number of benign lesions is equal to or greater than the threshold value TH, the display control unit 48A may perform control to display, on the display 23, information indicating the presence of a malignant lesion. Thereby, as a result of the user being able to ascertain the presence of a malignant lesion in the medical image, it is possible to suppress overlooking of malignant lesions by the user due to the presence of a large number of benign lesions in the medical image.

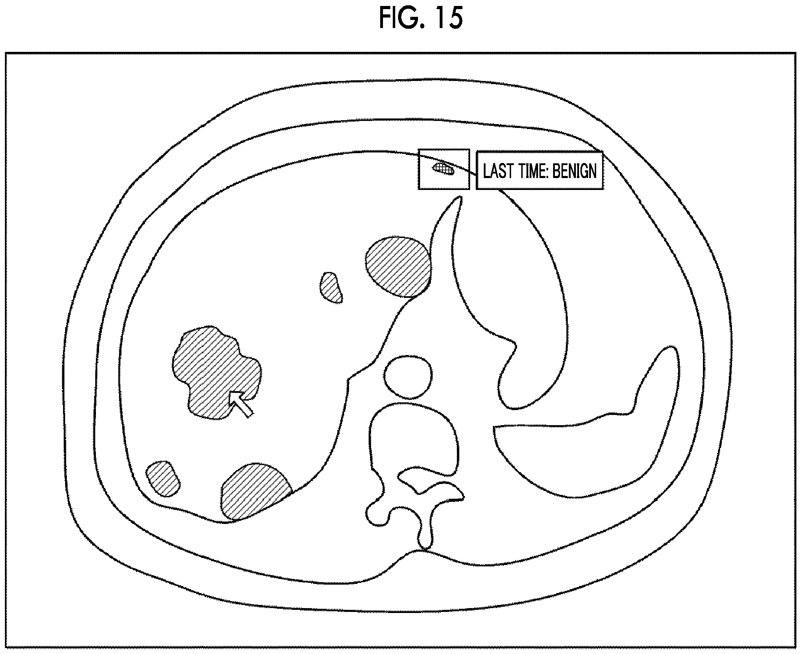

In addition, in a case where the attributes of the second lesion other than the first lesion selected by the selection unit 46 are different from the attributes detected in the past, the display control unit 48A may perform control to further display information indicating that the attributes are different. Specifically, as shown in Fig. 15 as an example, as described above, the display control unit 48A performs control to highlight a lesion having an attribute different from the attribute of the first lesion among the second lesions on the display 23. In this form example, the display control unit 48A performs control to further display information indicating that the attributes are different for the second lesion having the attributes different from the attributes detected in the past. Similarly to Fig. 9, Fig. 15 shows an example in which one of the benign lesions is designated by the user, and a lesion having an attribute different from the designated lesion, that is, a malignant lesion, was previously detected as benign. In addition, Fig. 15 shows an example in which text indicating that the lesion was benign in the examination at the last time is displayed as the information indicating that the attributes are different. In this form example, the user can ascertain that the lesion has changed from benign to malignant.

Next, with reference to Fig. 10, operations of the medical image apparatus 10 according to the present embodiment will be described. The CPU 20 executes the medical image program 30, whereby a lesion display process shown in Fig. 10 is executed. The lesion display process shown in Fig. 10 is executed, for example, in a case where an instruction to start execution is input by the user. Steps in Fig. 10 that execute the same processing as in Fig. 7 are given the same step numbers and descriptions thereof will be omitted.

In Step S14A of Fig. 10, as described above, the analysis unit 44A analyzes each of the lesions extracted in Step S12, and derives whether the lesion is benign or malignant.

In Step S20A, as described above, the display control unit 48A performs control to highlight, on the display 23, the lesion having an attribute different from the attribute of the first lesion selected in Step S18 among the second lesions. In a case where the process of Step S20A ends, the lesion display process ends.

As described above, according to the present embodiment, it is possible to appropriately support the creation of the medical document even in a case where the medical image includes a large number of regions of interest.

### [Third embodiment]

A third embodiment of the disclosed technology will be described. Since the configuration of the medical information system 1 and the hardware configuration of the medical image apparatus 10 according to the present embodiment are the same as those of the first embodiment, the description thereof will be omitted.

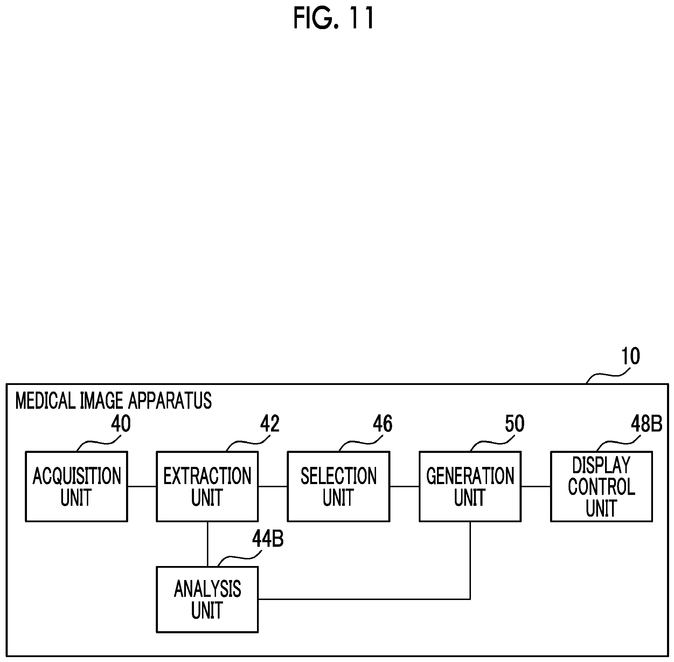

A functional configuration of the medical image apparatus 10 according to the present embodiment will be described with reference to Fig. 11. The same reference numerals are assigned to the functional units having the same functions as the medical image apparatus 10 according to the first embodiment, and the description thereof will be omitted. As shown in Fig. 11, the medical image apparatus 10 includes an acquisition unit 40, an extraction unit 42, an analysis unit 44B, a selection unit 46, a display control unit 48B, and a generation unit 50. The CPU 20 executes the medical image program 30 to function as the acquisition unit 40, the extraction unit 42, the analysis unit 44B, the selection unit 46, the display control unit 48B, and the generation unit 50.

The analysis unit 44B analyzes each of the lesions extracted by the extraction unit 42, and derives a name of the lesion as an example of attributes of the lesion. Further, the analysis unit 44B analyzes each of the lesions extracted by the extraction unit 42, and derives a finding of the lesion. In the following, in order to make the description easy to understand, an example in which the size is applied as a finding will be described. Examples of the size of the lesion include a major axis of the lesion.

Specifically, the analysis unit 44B derives a name and a finding of the lesion using a trained model M4 for deriving the name and the finding of the lesion. The trained model M4 is configured by, for example, a CNN that receives, for example, a medical image including a lesion and information specifying a region in the medical image in which the lesion is present as inputs, and outputs a name and a finding of the lesion. The trained model M4 is, for example, a model trained by machine learning using, as training data, a large number of combinations of a medical image including a lesion, information specifying a region in the medical image in which the lesion is present, and a name and a finding of the lesion.

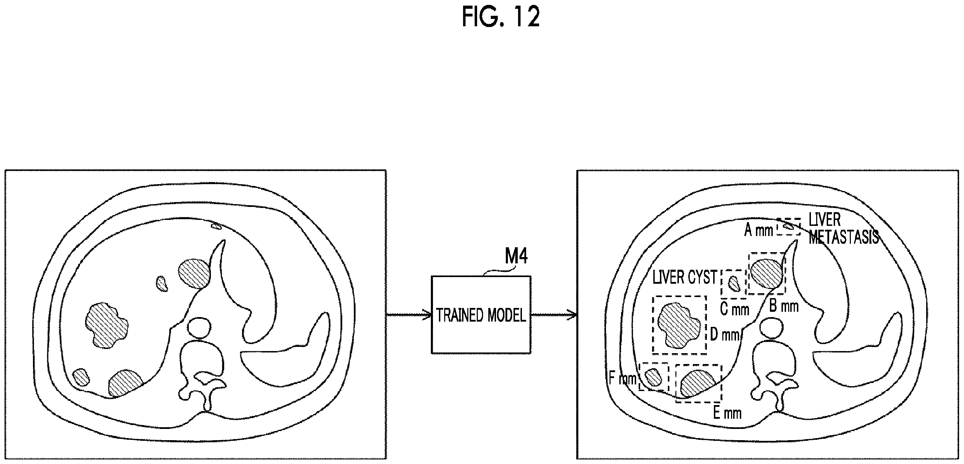

As shown in Fig. 12 as an example, the analysis unit 44B inputs, to the trained model M4, information specifying a diagnosis target image and a region in which a lesion extracted by the extraction unit 42 for the diagnosis target image is present. The trained model M4 outputs the name and the finding of the lesion included in the input diagnosis target image. Fig. 12 shows an example in which the name of five lesions is liver cyst and the name of one lesion is liver metastasis. Fig. 12 also shows the size derived for each lesion.

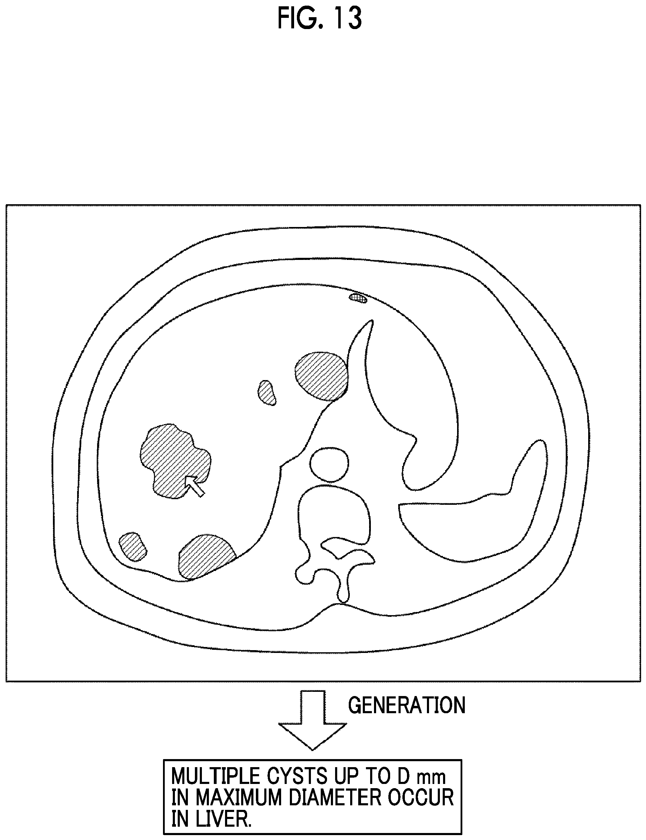

As shown in Fig. 13 as an example, the generation unit 50 generates a comment on findings summarizing the findings of a lesion having the same name as the name of the lesion selected by the selection unit 46. Fig. 13 shown an example in which one of lesions of five liver cysts (in the example of Fig. 13, the lesion pointed to by the arrow indicating the mouse pointer) is designated by the user, and a comment on findings summarizing the findings of the five liver cysts having the same name as the name of the designated lesion is generated.

For example, the generation unit 50 generates a comment on findings by inputting the name of the lesion selected by the selection unit 46 and the findings of the lesion having the same name as the name to a recurrent neural network trained to generate text from the input words.

Similarly to the display control unit 48 according to the first embodiment, the display control unit 48B performs control to display information indicating the plurality of lesions extracted by the extraction unit 42 on the display 23. In addition, the display control unit 48B performs control to display the comment on findings generated by the generation unit 50 on the display 23.

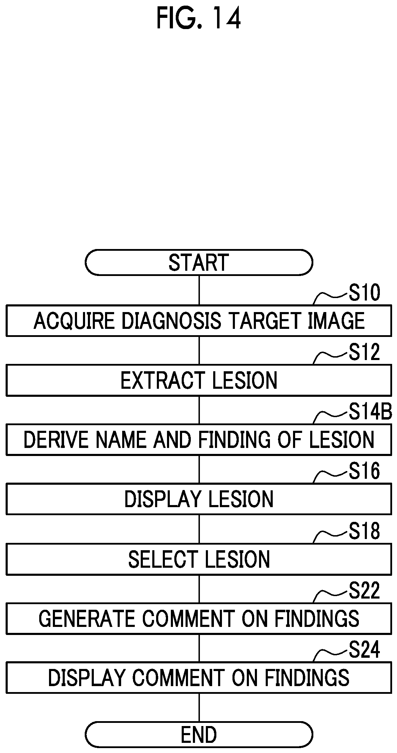

Next, with reference to Fig. 14, operations of the medical image apparatus 10 according to the present embodiment will be described. The CPU 20 executes the medical image program 30, whereby a comment-on-findings generation process shown in Fig. 14 is executed. The comment-on-findings generation process shown in Fig. 14 is executed, for example, in a case where an instruction to start execution is input by the user. Steps in Fig. 14 that execute the same processing as in Fig. 7 are given the same step numbers and descriptions thereof will be omitted.

In Step S14B of Fig. 14, as described above, the analysis unit 44B analyzes each of the lesions extracted in Step S12, and derives a name and a finding of the lesion.

In Step S22, as described above, the generation unit 50 generates a comment on findings summarizing the findings of the lesion having the same name as the name of the lesion selected in Step S18. In Step S24, the display control unit 48B performs control to display the comment on findings generated in Step S22 on the display 23. In a case where the process of Step S24 ends, the comment-on-findings generation process ends.

As described above, according to the present embodiment, it is possible to appropriately support the creation of the medical document even in a case where the medical image includes a large number of regions of interest.

Note that, in each of the above-described embodiments, for example, as a hardware structure of a processing unit that executes various kinds of processing, such as each functional unit of the medical image apparatus 10, the following various processors can be used. As described above, the various processors include a programmable logic device (PLD) as a processor of which the circuit configuration can be changed after manufacture, such as a field programmable gate array (FPGA), a dedicated electrical circuit as a processor having a dedicated circuit configuration for executing specific processing such as an application specific integrated circuit (ASIC), and the like, in addition to the CPU as a general-purpose processor that functions as various processing units by executing software (programs).

One processing unit may be configured by one of the various processors, or may be configured by a combination of the same or different kinds of two or more processors (for example, a combination of a plurality of FPGAs or a combination of the CPU and the FPGA). In addition, a plurality of processing units may be configured by one processor.

As an example in which a plurality of processing units are configured by one processor, first, there is a form in which one processor is configured by a combination of one or more CPUs and software as typified by a computer, such as a client or a server, and this processor functions as a plurality of processing units. Second, there is a form in which a processor for realizing the function of the entire system including a plurality of processing units via one integrated circuit (IC) chip as typified by a system on chip (SoC) or the like is used. In this way, various processing units are configured by one or more of the above-described various processors as hardware structures.

Furthermore, as the hardware structure of the various processors, more specifically, an electrical circuit (circuitry) in which circuit elements such as semiconductor elements are combined can be used.

In each of the above embodiments, the medical image program 30 has been described as being stored (installed) in the storage unit 22 in advance; however, the present disclosure is not limited thereto. The medical image program 30 may be provided in a form recorded in a recording medium such as a compact disc read only memory (CD-ROM), a digital versatile disc read only memory (DVD-ROM), and a universal serial bus (USB) memory. In addition, the medical image program 30 may be configured to be downloaded from an external device via a network.

The disclosures of Japanese Patent Application No. 2021-065375 filed on April 7, 2021 and Japanese Patent Application No. 2021-208525 filed on December 22, 2021 are incorporated herein by reference in their entirety. In addition, all literatures, patent applications, and technical standards described herein are incorporated by reference to the same extent as if the individual literature, patent applications, and technical standards were specifically and individually stated to be incorporated by reference.

## Claims

1. A medical image apparatus comprising:
at least one processor,
wherein the processor is configured to:
acquire a medical image, information indicating a plurality of regions of interest included in the medical image, and an attribute of each of the plurality of regions of interest;
select at least one region of interest from among the plurality of regions of interest; and
perform control to display information regarding a region of interest other than the selected region of interest based on an attribute of the selected region of interest.

2. The medical image apparatus according to claim 1,
wherein the processor is configured to
perform control to display information regarding a region of interest having the same attribute as the attribute of the selected region of interest.

3. The medical image apparatus according to claim 1,
wherein the processor is configured to
perform control to display information regarding a region of interest having an attribute different from the attribute of the selected region of interest.

4. The medical image apparatus according to claim 3,
wherein the processor is configured to,
in a case where the number of regions of interest having the same attribute as the attribute of the selected region of interest is equal to or greater than a threshold value, perform control to display the information regarding the region of interest having the attribute different from the attribute of the selected region of interest.

5. The medical image apparatus according to claim 4,
wherein the region of interest is a region including a lesion,
the attribute includes whether the lesion is benign or malignant, and
the processor is configured to,
in a case where the selected region of interest includes a benign lesion and the number of regions of interest including the benign lesion is equal to or greater than the threshold value, perform control to display information regarding a region of interest including a malignant lesion.

6. The medical image apparatus according to any one of claims 2 to 5,
wherein the processor is configured to
perform control to highlight the region of interest as the control to display the information regarding the region of interest.

7. The medical image apparatus according to any one of claims 3 to 5,
wherein the processor is configured to
perform control to display information indicating a presence of the region of interest having the attribute different from the attribute of the selected region of interest as the control to display the information regarding the region of interest.

8. The medical image apparatus according to any one of claims 1 to 7,
wherein the processor is configured to,
in a case where an attribute of the region of interest other than the selected region of interest is different from an attribute detected in the past, perform control to further display information indicating that the attributes are different.

9. A medical image method executed by a processor provided in a medical image apparatus, the method comprising:
acquiring a medical image, information indicating a plurality of regions of interest included in the medical image, and an attribute of each of the plurality of regions of interest;
selecting at least one region of interest from among the plurality of regions of interest; and
performing control to display information regarding a region of interest other than the selected region of interest based on an attribute of the selected region of interest.

10. A medical image program for causing a processor provided in a medical image apparatus to execute:
acquiring a medical image, information indicating a plurality of regions of interest included in the medical image, and an attribute of each of the plurality of regions of interest;
selecting at least one region of interest from among the plurality of regions of interest; and
performing control to display information regarding a region of interest other than the selected region of interest based on an attribute of the selected region of interest.

11. A medical image apparatus comprising:
at least one processor,
wherein the processor is configured to:
acquire a medical image, information indicating a plurality of regions of interest included in the medical image, and an attribute of each of the plurality of regions of interest;
select at least one region of interest from among the plurality of regions of interest; and
generate a comment on findings for a region of interest having the same attribute as an attribute of the selected region of interest.

12. A medical image method executed by a processor provided in a medical image apparatus, the method comprising:
acquiring a medical image, information indicating a plurality of regions of interest included in the medical image, and an attribute of each of the plurality of regions of interest;
selecting at least one region of interest from among the plurality of regions of interest; and
generating a comment on findings for a region of interest having the same attribute as an attribute of the selected region of interest.

13. A medical image program for causing a processor provided in a medical image apparatus to execute:
acquiring a medical image, information indicating a plurality of regions of interest included in the medical image, and an attribute of each of the plurality of regions of interest;
selecting at least one region of interest from among the plurality of regions of interest; and
generating a comment on findings for a region of interest having the same attribute as an attribute of the selected region of interest.
